# EUROPEAN PATENT SPECIFICATION

(11) **EP 4 141 412 B1**
(45) Date of publication and mention of the grant of the patent: **26.08.2026**
(21) Application number: 21792824.1
(22) Date of filing: 19.04.2021
(51) Int. Cl.: G01N 15/10, G16H 50/20, G01N 15/1429

(54) **HEMATOLOGICAL PARAMETER FOR VIRAL INFECTION**
HÄMATOLOGISCHER PARAMETER FÜR VIRUSINFEKTION
PARAMÈTRE HÉMATOLOGIQUE POUR UNE INFECTION VIRALE

(30) Priority: 21.04.2020 CN 202010319750
(43) Date of publication of application: 01.03.2023
(73) Proprietor: Beckman Coulter, Inc., Brea, CA 92821 (US)
(72) Inventor: DU, Yuxiao, Beijing 100029 (CN)
(74) Representative: Maiwald GmbH
(86) International application number: PCT/CN2021/087984
(87) International publication number: WO 2021/213291

(56) References cited:
- CN-A- 108 604 466
- CN-A- 110 031 614
- FRATER JOHN L. ET AL: "COVID-19 and the clinical hematology laboratory", vol. 42, no. S1, 20 April 2020 (2020-04-20), GB; US, pages 11 - 18, XP055816538, ISSN: 1751-5521, Retrieved from the Internet <URL:https://onlinelibrary.wiley.com/doi/full-xml/10.1111/ijlh.13229> [retrieved on 20240327], DOI: 10.1111/ijlh.13229
- RAI SHARADA ET AL: "Utility of DxH 800 VCS Parameters and Lymph Index in Predicting Dengue", 1 January 2019 (2019-01-01), XP093146184, ISSN: 2249-782X, Retrieved from the Internet <URL:https://www.researchgate.net/publication/335162766_Utility_of_DxH_800_VCS_Parameters_and_Lymph_Index_in_Predicting_Dengue/fulltext/5e7b577f92851cdfca2fa43f/Utility-of-DxH-800-VCS-Parameters-and-Lymph-Index-in-Predicting-Dengue.pdf> [retrieved on 20240327], DOI: 10.7860/JCDR/2019/38500.13044
- ZHU Y. ET AL: "The lymph index: a potential hematological parameter for viral infection", INTERNATIONAL JOURNAL OF INFECTIOUS DISEASES, vol. 17, no. 7, 1 July 2013 (2013-07-01), CA, pages e490 - e493, XP093146167, ISSN: 1201-9712, DOI: 10.1016/j.ijid.2012.12.002
- YAN, YUFEI ET AL.: "Role of Neutrophil/Lymphocyte Ratio in Covid-19 (online first)", LABORATORY MEDICINE, 14 April 2020 (2020-04-14), pages 1 - 7, XP009531373, ISSN: 1673-8640

## Description

### FIELD

The present invention relates to the detection of Coronavirus infections.

### BACKGROUND

In December 2019, a pneumonia epidemic of unknown cause broke out in Wuhan, China. Subsequent deep sequencing analysis of lower respiratory tract samples showed that the pathogen was a new coronavirus [1].

Coronavirus, belonging to the Nidovirales, Coronaviridae, Coronavirus genus, is a type of RNA virus with an envelope and a linear single-stranded positive strand as the genome, and is a large class of viruses widely existing in nature. The genome of Coronavirus has a methylated cap structure at the 5 'end and a poly (A) tail at the 3' end, and a full-length of about 27-32 kb, which is the largest among the genome of known RNA viruses. Six subtypes of Coronaviruses (HCoV-229E, HCoV-OC43, SARS-CoV, HCoV-NL63, HCoV-HKU1, and MERS-CoV) have been known to infect humans. Among them, 229E, NL63, OC43 and HKU1 usually cause mild or moderate upper respiratory tract diseases such as cold, while SARS-CoV and MERS-CoV can cause serious fatal diseases. The pathogen causing the outbreak of pneumonia in Wuhan is the seventh subtypes of Coronavirus confirmed to infect humans. Studies have shown that its genetic structure is 82% similar to SARS-CoV [2]. Based on this similarity, the International Committee on Taxonomy of Viruses (ICTV) named the virus as Severe Acute Respiratory Syndrome Coronavirus Type 2 (SARS-CoV-2), and the World Health Organization (WHO) named the pneumonia disease as Coronavirus Disease 2019 (COVID-19).

Currently, COVID-19 has been declared a global pandemic by WHO and has spread to more than 100 countries. As of March 2020, the total number of patients in the world has reached 900,000, the number of deaths has exceeded 40,000, and these numbers are still increasing rapidly. Recent studies revealed that SARS-CoV-2 could transmit among human, mainly through respiratory droplets [3]. The incubation period is generally 3-7 days, up to 14 days. The main symptoms (low fever, fatigue, and dry cough) are usually mild and non-specific, which make it difficult to identify infected individuals early or in the incubation period.

Although several laboratory tests, such as lymphocyte count, C-reactive protein, chest imaging studies or molecular testing [4], have been used to aid diagnosis of Coronavirus, they are neither specific nor sensitive or not widely available. At present, the diagnostic standard commonly used in various countries is to perform nucleic acid specific detection on patient samples (such as nasal swabs), but such method generally has the disadvantages of poor repeatability, insufficient sensitivity (especially in the early stage of infection), inaccurate sampling, and high requirements for technical personnel.

From an epidemiological point of view, it is important to identify infected individuals with high sensitivity early in the infection. Especially for individuals who are in close contact with patients with known COVID-19 or who are in epidemic areas, early screening and diagnosis can provide valuable information for timely and effective medical observation and intervention, triage decisions, and even control decisions in disease endemic areas. In the current diagnosis of COVID-19, a patient with the contact history, clinical symptoms, CT image resembling viral infection and positive molecular testing is generally determined as a confirmed case, while whose with contact history, clinical symptoms, CT image resembling viral infection and negative molecular testing on admission are determined as suspected cases. The disadvantage is that there is a lag in the detection of nucleic acid molecules (an important marker of COVID-19 diagnosis), which is not desirable for early screening of the disease. The reason may be that the viral load in the early stage of the disease is low, making it difficult to obtain high-sensitivity results through nucleic acid molecule detection. This makes it difficult to carry out early identification, classification, and therapeutic intervention for "suspected" individuals, and even leads to the further spread of the disease due to lack of timely isolation. Therefore, there is an urgent need to find a way to quickly identify patients who may be infected, especially in the early stages of infection, so as to provide valuable information for triage decisions.

FRATER JOHN L. ET AL: "COVID-19 and the clinical hematology laboratory",INTERNATIONAL JOURNAL OF LABORATORY HEMATOLOGY,vol. 42, no. S1 20 April 2020 (2020-04-20), pages 11-18, discloses a prior art method for identifying a subject having a viral infection, wherein the viral infection is Coronavirus infection.

### SUMMARY

The invention is defined by a method, an apparatus and a computer storage media according to the respective independent claims 1, 7 and 12. Further aspects are detailed in the dependent claims.

Modern automated hematology analyzer with VCS (volume, conductivity, and light scatter) technology can determine the intrinsic biophysical properties of over 8000 peripheral leukocytes in their 'near in vivo state'. VCS technology uses direct current impedance for the cell volume, radio frequency opacity to evaluate conductivity for cytoplasmic chemical composition and nuclear volume, and a laser beam to measure multiple angles of light scatter for cell surface topography, cytoplasmic granularity and nuclear structure. It can also measure the degree of cell volume variation. These morphometric measurements are known as cell population data (CPD). The inventor found that, dynamic changes in volumetric parameters of circulating activated mononuclear cells in response to SARS-CoV-2 infection could be quantitatively determined by automated hematology analyzer and would serve as useful biomarkers for rapidly screening those suspected individuals who have been in close contact with known COVID-19 patients or in epidemic region for likely infection.

The present disclosure relates to volume biomarkers for early diagnosis of Coronavirus infections, such as monocyte distribution width (MDW), lymphocyte volume (LV), lymphocyte distribution width (LV-SD), lymphocyte conductivity (LC), lymph Index, mean neutrophil volume (MNV), neutrophil distribution width (NDW). The invention also relates to a method, device and computer executable program for the early diagnosis of Coronavirus infection using such biomarkers. According to some technical solutions of the present invention, the automated volume parameter lymph index and MDW can be used as viral biomarkers to help healthcare workers in the out-patient department or fever clinic to rapidly identify those who might be infected with COVID-19 and to provide valuable information for triage decision making.

In the study, the inventors surprisingly found that for those patients suspected of early COVID-19 who have not been diagnosed, the dynamic changes in volumetric parameters of circulating activated mononuclear cells in response to SARS-CoV-2 infection could be quantitatively determined by automated hematology analyzer and would serve as useful biomarkers for rapidly screening. The inventors demonstrated that some cell population data, especially the lymph index and monocyte distribution width (MDW) were significantly increased in COVID-19 patients. With designated cutoff values for lymph index and MDW, we achieved the sensitivities of 84.4% and 78.1%, respectively in diagnosing COVID-19 infection. In addition, lymph index in combination with MDW demonstrates excellent diagnostic performance (AUC of 0.83, higher that the AUC of 0.77 with MDW alone and the AUC of 0.79 with lymph index alone). This shows that the combination of MDW and lymph index can more truly reflect the possibility of the subject suffering from SARS-CoV-2 infection, compared with MDW or lymph index alone. Furthermore, we show that lymph index and MDW in suspected patients, like confirmed patients were also significantly increased at the time of admission, suggesting similar pathophysiological process. In particular, 89% of the suspected patients with positive test according to this protocol (with negative nucleic acid molecule test) were diagnosed with COVID-19 during the subsequent disease course. This illustrates clinical significance that lymph index and MDW can be used as sensitive screening biomarkers to rapidly identify those suspected individuals before nuclear acid confirmation and to develop proper management plan.

One aspect of the present disclosure relates to a method for identifying a subject having a viral infection, specifically, according to the claims a Coronavirus infection, the method comprising:
1) flowing a body fluid sample obtained from the subject through a flowcell;
2) measuring individual cells of the plurality of cells in the body fluid sample; according to the claims, the measuring comprises measuring one or more of volume parameter, conductivity parameter, and light scatter parameter, or light scatter and direct current impedance;
3) determining one or more cell population parameter data values in the body fluid sample based on the measuring, wherein the one or more cell population parameter data values comprise a population parameter data value from one or more of lymphocyte, neutrophil and monocyte; according to the claims, the cell population data comprises monocyte distribution width (MDW) and lymph index; and
4) determining whether or not at least one of the one or more cell population data values exceeds a predetermined threshold,
   wherein if at least one of the one or more cell population parameter values exceeds a predetermined threshold, the viral infection in the subject is indicated. According to the claims, if the values of the MDW and the lymph index are both outside of their respective reference ranges, the Coronavirus infection in the subject is indicated. According to the claims, the lymph index is calculated according to: lymph index = lymphocyte volume (LV) × lymphocyte distribution width (LV-SD) / lymphocyte conductivity (LC).

In an example of this aspect, the viral infection is associated with upper respiratory tract illnesses. According to the claims, the viral infection is Coronavirus infection. In an embodiment, the Coronavirus is SARS-CoV-2. In an embodiment, the upper respiratory tract illness is COVID-19.

According to the claims, the cell population data comprises monocyte distribution width (MDW) and lymph index. According to the present disclosure, the cell population data may comprise one or more selected from the group consisting of monocyte distribution width (MDW), lymphocyte volume (LV), lymphocyte distribution width (LV-SD), lymphocyte conductivity (LC), lymph index, mean neutrophil volume (MNV), neutrophil distribution width (NDW), or any combinations thereof. In an example of the present disclosure, the cell population data comprises MDW, and one or more of lymph index and LV-SD. In an example of the present disclosure, the cell population data comprises a combination of two or more selected from the group consisting of MDW, LV, LV-SD, LC, and lymph index. In an example of the present disclosure, the cell population data is a combination of MDW and one or more of LV, LV-SD, LC and lymph index. In an embodiment, the cell population data consists of MDW and lymph index. In an example of the present disclosure, the cell population data comprises or consists of MNV and NDW. The lymph index is calculated according to lymph index = LV × (LV-SD) / LC.

In an embodiment, if one or more of the cell population data exceeds a predetermined threshold, the viral infection in the subject is indicated.

In an embodiment, if the value of the MDW is greater than 20.27, the viral infection in the subject is indicated. In a preferred embodiment, if the value of the MDW is greater than 20.42, the viral infection in the subject is indicated. In a more preferred embodiment, if the value of the MDW is greater than 20.73, the viral infection in the subject is indicated. In an even more preferred embodiment, if the value of the MDW is greater than 21.24, the viral infection in the subject is indicated. In an embodiment, if the value of the lymph index is greater than 11.3, the viral infection in the subject is indicated. In a preferred embodiment, if the value of the lymph index is greater than 11.63, the viral infection in the subject is indicated. In a more preferred embodiment, if the value of the lymph index is greater than 11.8, the viral infection in the subject is indicated. In an even more preferred embodiment, if the value of the lymph index is greater than 12.04, the viral infection in the subject is indicated. In an even more preferred embodiment, if the value of the lymph index is greater than 12.35, the viral infection in the subject is indicated. In examples of the present disclosure, if the value of LV-SD is greater than 14.41, the viral infection in the subject is indicated.

In an embodiment, the body fluid sample is whole blood.

In an embodiment, the measuring comprises measuring one or more of volume parameter, conductivity parameter, and/or light scatter parameter. In an embodiment, the measuring comprises measuring light scatter and direct current impedance from individual cells of the plurality of cells.

In an embodiment, the subject is an individual not confirmed of viral infection. In a preferred embodiment, the subject is an individual suspected of viral infection. In an embodiment, the subject is negative for viral nuclear acid testing. In an embodiment, the subject is human.

Another aspect of the present disclosure relates to a device for identifying a subject having a viral infection, specifically, according to the claims a Coronavirus infection, the device comprising:
1) a transducer for measuring cells passing through a flowcell; according to the claims, the measuring comprises measuring one or more of volume parameter, conductivity parameter, and light scatter parameter, or light scatter and direct current impedance from the cells; and
2) one or more processors configured to perform operations comprising:
   receiving and processing measurement data from the transducer,
   determining one or more cell population data values based on the measuring, wherein the one or more cell population parameter data values comprise a population parameter data value from one or more of lymphocyte, neutrophil and monocyte; according to the claims, the cell population data comprises monocyte distribution width (MDW) and lymph index, and
   determining whether or not at least one of the one or more cell population data values exceeds a predetermined threshold,
   wherein if at least one of the one or more cell population parameter values exceeds a predetermined threshold, the viral infection in the subject is indicated. According to the claims, if the values of the MDW and the lymph index are both outside of their respective reference ranges, the Coronavirus infection in the subject is indicated. According to the claims, the lymph index is calculated according to: lymph index = lymphocyte volume (LV) × lymphocyte distribution width (LV-SD) / lymphocyte conductivity (LC).

In an example of this aspect, the viral infection is associated with upper respiratory tract illnesses. According to the claims, the viral infection is Coronavirus infection. In an embodiment, the Coronavirus is SARS-CoV-2. In an embodiment, the upper respiratory tract illness is COVID-19.

According to the claims, the cell population data comprises monocyte distribution width (MDW) and lymph index. According to the present disclosure, the cell population data may comprise one or more selected from the group consisting of monocyte distribution width (MDW), lymphocyte volume (LV), lymphocyte distribution width (LV-SD), lymphocyte conductivity (LC), lymph index, mean neutrophil volume (MNV), neutrophil distribution width (NDW), or any combinations thereof. In an example of the present disclosure, the cell population data comprises MDW, and one or more of lymph index and LV-SD. In an example of the present disclosure, the cell population data comprises a combination of two or more selected from the group consisting of MDW, LV, LV-SD, LC, and lymph index. In an example of the present disclosure, the cell population data is a combination of MDW and one or more of LV, LV-SD, LC and lymph index. In an embodiment, the cell population data consists of MDW and lymph index. In an example of the present disclosure, the cell population data comprises or consists of MNV and NDW. The lymph index is calculated according to lymph index = LV × (LV-SD) / LC.

According to the present disclosure, if the values of the MDW and the lymph index are both outside of their respective reference ranges, the viral infection in the subject is indicated. According to the claims, if the values of the MDW and the lymph index are both outside of their respective reference ranges, the Coronavirus infection in the subject is indicated.

In an embodiment, if the value of the MDW is greater than 20.27, the viral infection in the subject is indicated. In a preferred embodiment, if the value of the MDW is greater than 20.42, the viral infection in the subject is indicated. In a more preferred embodiment, if the value of the MDW is greater than 20.73, the viral infection in the subject is indicated. In an even more preferred embodiment, if the value of the MDW is greater than 21.24, the viral infection in the subject is indicated. In an embodiment, if the value of the lymph index is greater than 11.3, the viral infection in the subject is indicated. In a preferred embodiment, if the value of the lymph index is greater than 11.63, the viral infection in the subject is indicated. In a more preferred embodiment, if the value of the lymph index is greater than 11.8, the viral infection in the subject is indicated. In an even more preferred embodiment, if the value of the lymph index is greater than 12.04, the viral infection in the subject is indicated. In an even more preferred embodiment, if the value of the lymph index is greater than 12.35, the viral infection in the subject is indicated. In examples of the present disclosure, if the value of LV-SD is greater than 14.41, the viral infection in the subject is indicated.

In an embodiment, the measuring comprises measuring one or more of volume parameter, conductivity parameter, and/or light scatter parameter. In an embodiment, the measuring comprises measuring light scatter and direct current impedance from the cells.

In an embodiment, the subject is an individual not confirmed of viral infection. In a preferred embodiment, the subject is an individual suspected of viral infection. In an embodiment, the subject is negative for viral nuclear acid testing. In an embodiment, the subject is human.

Yet another aspect of the present disclosure relates to a computer storage media recording an executable program for identifying a subject having a viral infection, specifically, according to the claims a Coronavirus infection, characterized in that when the executable program is executed by a processor, the following steps are performed:
1) measuring cells passing through a flowcell; according to the claims, the measuring comprises measuring one or more of volume parameter, conductivity parameter, and light scatter parameter, or light scatter and direct current impedance from the cells;
2) determining one or more cell population parameter data values in the body fluid sample based on the measuring, wherein the one or more cell population parameter data values comprise a population parameter data value from one or more of lymphocyte, neutrophil and monocyte; according to the claims, the cell population data comprises monocyte distribution width (MDW) and lymph index; and
3) determining whether or not at least one of the one or more cell population data values exceeds a predetermined threshold,
   wherein if at least one of the one or more cell population parameter values exceeds a predetermined threshold, the viral infection in the subject is indicated. According to the claims, if the values of the MDW and the lymph index are both outside of their respective reference ranges, the Coronavirus infection in the subject is indicated. According to the claims, the lymph index is calculated according to: lymph index = lymphocyte volume (LV) × lymphocyte distribution width (LV-SD) / lymphocyte conductivity (LC).

In an example of this aspect, the viral infection is associated with upper respiratory tract illnesses. According to the claims, the viral infection is Coronavirus infection. In an embodiment, the Coronavirus is SARS-CoV-2. In an embodiment, the upper respiratory tract illness is COVID-19.

According to the claims, the cell population data comprises monocyte distribution width (MDW) and lymph index. According to the present disclosure, the cell population data may comprise one or more selected from the group consisting of monocyte distribution width (MDW), lymphocyte volume (LV), lymphocyte distribution width (LV-SD), lymphocyte conductivity (LC), lymph index, mean neutrophil volume (MNV), neutrophil distribution width (NDW), or any combinations thereof. In an example of the present disclosure, the cell population data comprises MDW, and one or more of lymph index and LV-SD. In an example of the present disclosure, the cell population data comprises a combination of two or more selected from the group consisting of MDW, LV, LV-SD, LC, and lymph index. In an example of the present disclosure, the cell population data is a combination of MDW and one or more of LV, LV-SD, LC and lymph index. In an embodiment, the cell population data consists of MDW and lymph index. In an example of the present disclosure, the cell population data comprises or consists of MNV and NDW. The lymph index is calculated according to lymph index = LV × (LV-SD) / LC.

In an embodiment, if one or more of the cell population data exceeds a predetermined threshold, the viral infection in the subject is indicated.

In an embodiment, if the value of the MDW is greater than 20.27, the viral infection in the subject is indicated. In a preferred embodiment, if the value of the MDW is greater than 20.42, the viral infection in the subject is indicated. In a more preferred embodiment, if the value of the MDW is greater than 20.73, the viral infection in the subject is indicated. In an even more preferred embodiment, if the value of the MDW is greater than 21.24, the viral infection in the subject is indicated. In an embodiment, if the value of the lymph index is greater than 11.3, the viral infection in the subject is indicated. In a preferred embodiment, if the value of the lymph index is greater than 11.63, the viral infection in the subject is indicated. In a more preferred embodiment, if the value of the lymph index is greater than 11.8, the viral infection in the subject is indicated. In an even more preferred embodiment, if the value of the lymph index is greater than 12.04, the viral infection in the subject is indicated. In an even more preferred embodiment, if the value of the lymph index is greater than 12.35, the viral infection in the subject is indicated. In examples of the present disclosure, if the value of LV-SD is greater than 14.41, the viral infection in the subject is indicated.

In an embodiment, the measuring comprises measuring one or more of volume parameter, conductivity parameter, and/or light scatter parameter. In an embodiment, the measuring comprises measuring light scatter and direct current impedance from the cells.

In an embodiment, the subject is an individual not confirmed of viral infection. In a preferred embodiment, the subject is an individual suspected of viral infection. In an embodiment, the subject is negative for viral nuclear acid testing. In an embodiment, the subject is human.

### DEFINITION

Some terms used herein are defined as follows. These parameters can be easily measured using commercially available instruments (such as UniCel DxH 800 Hematology Analyzer (Beckman Coulter, Brea, CA)).

| **Parameter** | **Full name** | **Quantitative method** |
|---|---|---|
| MDW | monocyte distribution width | Standard deviation (SD) of monocyte volume value |
| MMV | mean monocyte volume | Mean (mean) of monocyte volume value |
| MNV | mean neutrophil volume | Mean (mean) of neutrophil volume value |
| NDW | neutrophil distribution width | Standard deviation (SD) of neutrophil volume value |
| LV | lymphocyte volume | Mean (mean) of lymphocyte volume value |
| LV-SD | lymphocyte distribution width | Standard deviation (SD) of lymphocyte volume value |
| LC | lymphocyte conductivity | Mean (mean) of lymphocyte conductivity value |
| lymph index | lymph index | lymph index = LV × (LV-SD)/LC |
| WBC | White blood cell | Measured value of the instrument |
| NE | Neutrophil | Measured value of the instrument |
| LY | Lymphocyte | 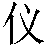 Measured value of the instrument |
| MO | Monocyte | Measured value of the instrument |
| NLR | Neutrophil/lymphocyte ratio | N#/L# |

### BRIEF DESCRIPTION OF DRAWINGS

Figure 1 shows the distribution of MDW and lymph index among the three groups (control, suspected, and confirmed). Figure 1a shows the distribution of lymph index, and Figure 2a shows the distribution of MDW.
Figure 2 shows the results of using CPD parameters to predict the diagnosis of SARS-CoV-2 infection. Among them, the control (n = 32) is relative to the confirmed diagnosis (n = 68).

### DETAILED DESCRIPTION

### Examples

### Materials and Method

### Case selection and data collection

In this case-control study, clinical information including contact history, initial symptoms, routine hematology analysis, chest CT and RT-PCT analysis was collected from a total 128 hospitalized patients of Chinese ethnicity from Feb 14 to 29, 2020 in The Wuhan Union Hospital, Wuhan China. Based on the criteria complied with the guideline for the diagnosis and treatment of 2019 novel coronavirus (COVID-19) infected pneumonia (sixth version), there were 96 patients (male: female ratio, 40:56) including 68 confirmed cases (contact history, clinical symptoms, CT image resembling viral infection and positive molecular testing) and 28 suspected cases (contact history, clinical symptoms, CT image resembling viral infection and negative molecular testing on admission). 32 individuals without clinical and radiological evidence of viral infection were used as controls.

### Classification criteria

Based on the criteria complied with the guideline for the diagnosis and treatment of 2019 novel coronavirus (COVID-19) infected pneumonia (sixth version), the classification indicators are as follows:

### (I) Suspected cases

Comprehensive analysis combining the following epidemiological history and clinical manifestations:

### 1. Epidemiology history

(1) A travel history or residence history in Wuhan city and surrounding areas thereof, or a travel history or residence history in other communities with case reports within 14 days before onset of illness;
(2) A history of contact with a patient with new Coronavirus infection (positive nucleic acid testing) within 14 days before onset of illness;
(3) A history of contact with a patient with fever or respiratory symptoms from Wuhan and surrounding areas thereof, or from communities with reported cases within 14 days before onset of illness;
(4) Clustering occurrence of ill.

### 2. Clinical manifestations

(1) Fever and/or respiratory symptoms;
(2) Imaging features of new Coronavirus pneumonia;
(3) Normal or decreased total number of white blood cells, reduced lymphocyte count at the early stage of onset of illness.

Comply with both any one of the epidemiological history and any two of the clinical manifestations.

No clear epidemiological history, but comply with 3 of the clinical manifestations.

### (II) Confirmed cases

Suspected cases with one of the following etiological evidence:
1. Positive detection of new coronavirus nucleic acid by real-time fluorescent RT-PCR;
2. Highly homologous to known new coronaviruses by viral gene sequencing.

### Cell population data (CPD) analysis

All blood samples were analyzed on a UniCel DxH 800 hematology analyzer (Beckman Coulter, Brea, CA) with version 2.0 software within 4 h of collection. This instrument measures CBC with differentials as well as cell morphometric parameters including specific cell volume and distribution of cell volumes within a group of cells, such as mean neutrophil volume (MNV), neutrophil distribution width (NDW), mean monocyte volume (MMV), monocyte distribution width (MDW), mean lymphocyte volume (LV) and lymphocyte distribution width (LV-SD). A simplified lymphocyte CPD, the lymph index, was calculated as LV* LV-SD/LC (lymphocyte conductivity). In addition, 5-angle light-scattering parameters were also collected, including median-angle light scatter, upper median-angle light scatter, lower median-angle light scatter, low-angle light scatter, and axial light loss. The light scatter parameters numerically capture the morphological changes reflected by cellular complexity, granularity, and nuclear structure, with the measurement of AL2 reflecting cell size on the basis of absorbed light.

### Statistical analysis

All the analysis including ROC are performed with SAS software, the version is 9.4. Since there are total 13 variables for 3 groups for analysis. If the variables are normal distribution, we use one-way ANOVA to test the difference among 3 groups. If the variables are non-normal distribution, we use Kruskal-Wallis to test the difference among 3 groups. If the P-value is less than 0.05, we can conclude that there is a significant difference among 3 groups. Then we use Dwass-Steel-Critchlow-Fligner test for two pairwise comparisons of non-normal distribution variables, if the P-value is less than 0.05, we can conclude that there is a significant difference between 2 groups. In the end, we use Tukey's Studentized Range (HSD) test for two pairwise comparisons of normal distribution variables, if the P-value is less than 0.05, we can conclude that there is a significant difference between 2 groups.

### Results

### Demographic data

Epidemiological information, clinical data, laboratory tests, and radiological characteristics were reviewed from electronic medical records. According to guideline for the diagnosis and treatment of 2019 novel coronavirus (COVID-19) infected pneumonia (sixth version), we prospectively collected and analyzed data from 128 patients (mean age 48.9 years, range 16-88 years; male: female ratio 61:67). There were 68 confirmed cases (common type), 28 suspected cases and 32 cases without clinical and radiological evidence of viral infection as controls. Among 68 confirmed cases, the male to female was 32:36; 68% (46/68) had fever and 47% (32/68) had cough. Among 28 suspected cases, the male to female was 8:20; 68% (19/28) had fever and 57% (16/28) had cough. Note: On follow-up of those suspected patients, 89% (25/28) was subsequently confirmed by positive nuclear testing with COVID-19; 11% (3/28) remained suspicious for COVID-19 infection. This shows that the method of the present disclosure can accurately identify SARS-CoV-2 infected persons at an earlier stage.

### Comparison of conventional hematologic parameters and CPD

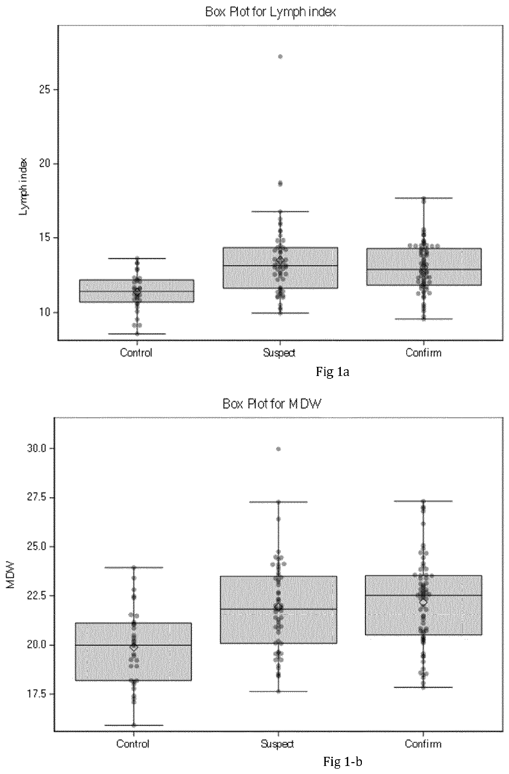

As shown in Table 1, no statistically significant differences in conventional hematologic parameters in terms of WBC, percent neutrophils, lymphocytes, monocytes and neutrophil/lymphocyte ratio were observed among all three groups. However, the monocyte distribution width (NDW), lymphocyte distribution width (LV-SD) and lymph index (LV* LV-SD/LC) were significantly increased in both suspected and confirmed group compared to those in the controls (Figure 1). No significant differences in NDW, LV-SD and lymph index were seen between suspected group and confirmed group. Although increase in mean lymphocyte volume (LV) and decrease in mean lymphocyte conductivity (LC) were also noted in both suspected and confirmed group, there were no statistically significant differences compared to the controls.

**Table 1 CBC & CPD parameter in three group patients**

| | **Control (n =32)** | | **Suspect (n=28)** | | **Confirm (n=68)** | |
|---|---|---|---|---|---|---|
| | **Mean±SD** | | **Mean±SD** | | **Mean±SD** | |
| MDW | 19.90± | 1.95 | 21.71± | 2.53 * | 22.16± | 2.26 * * |
| MMV | 170.91± | 8.45 | 174.39± | 7.96 | 174.19± | 9.10 |
| MNV | 146.47± | 6.51 | 145.29± | 5.28 | 143.78± | 5.98 |
| NDW | 18.62± | 3.24 | 17.61± | 2.53 | 17.16± | 1.15 |
| LV | 87.84± | 4.23 | 88.67± | 3.27 | 89.04± | 4.59 |
| LV-SD | 14.62± | 1.66 | 15.97± | 1.56 * | 16.25± | 1.71 * * |
| LC | 113.13± | 7.13 | 110.71± | 3.17 | 111.31± | 3.23 |
| Lymph-Index | 11.36± | 1.25 | 12.81± | 1.55 * | 13.02± | 1.68 * * |
| WBC | 6.54± | 2.65 | 5.96± | 2.61 | 5.65± | 1.84 |
| NE | 61.20± | 11.21 | 67.77± | 12.14 | 61.90± | 16.21 |
| LY | 25.29± | 9.62 | 20.66± | 10.34 | 24.89± | 11.61 |
| MO | 9.50± | 2.39 | 10.16± | 2.85 | 11.00± | 9.73 |
| NLR | 3.37± | 3.26 | 5.63± | 6.53 | 3.71± | 3.58 |

| | | | | | | |
|---|---|---|---|---|---|---|
| * Suspect vs Control, p <0.05 * * Confirm vs Control, p <0.05 | | | | | | |

### The sensitivity and the specificity of CPD in diagnosing COVID-19 infection

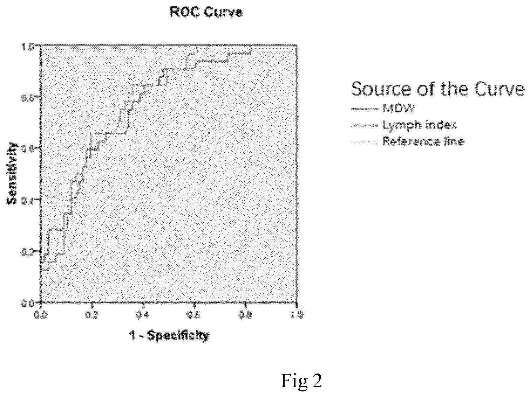

The sensitivity and the specificity of CPD in predicting COVID-19 infection were then calculated at designated cutoff values. As indicated in Table 2, the MDW and lymph index demonstrated the best sensitivity (78.1% and 84.4%, respectively) and specificity (64.2% and 64.2%, respectively) to detect COVID-19 infection compared to other parameters. ROC curves analysis revealed that MDW and lymph index had the largest areas under the curve (AUC) of 0.77 and 0.79, respectively. With combination of MDW and lymph index, the AUC increases to 0.83 (Figure 2, Table 2), higher than the AUC of MDW alone and the AUC of lymph index alone. This shows that the combination of MDW and lymph index can more truly reflect the possibility of the subject suffering from SARS-CoV-2 infection, compared with MDW or lymph index alone. In addition, due to different requirements for sensitivity and specificity in different application scenarios, for example, higher sensitivity is desired in the initial diagnosis and screening, and better specificity is needed in the diagnosis, we also conducted an in-depth study of the relationship between the cutoff point of MDW and lymph index and he sensitivity and specificity (Table 3). The results show that the method of the present disclosure can be better adapted to different application scenarios by adjusting the cutoff value.

**Table 2 CPD parameter for Predicting 2019-nCoV infection**

| | **AUC** | **Cutoff Point** | **Sensitivity,%** | **Specificity,%** |
|---|---|---|---|---|
| MDW | 0.772 | 20.27 | 79.41 | 59.38 |
| MMV | 0.607 | 172.5 | 65.6 | 58.2 |
| MNV | 0.397 | 144.5 | 40.6 | 51.5 |
| NDW | 0.321 | 17.23 | 34.4 | 42.4 |
| Lymph Index | 0.79 | 11.3 | 85.29 | 50 |
| NLR | 0.516 | 2.60 | 59.4 | 47.8 |
| MDW+ Lymph Index | 0.831 | | 85.3 | 53.1 |

| | | | | |
|---|---|---|---|---|
| *AUC, area under the receiver operating characteristic curve. | | | | |

**Table 3. Sensitivity and specificity of MDW and lymph index**

| | **AUC** | **Cutoff Point** | **Sensitivity,%** | **Specificity,%** |
|---|---|---|---|---|
| MDW | 0. 7730 | 20. 27 [20. 27, 20. 29) | 79.41 | 59.38 |
| MDW | 0. 7730 | 20. 42 [20. 42, 20.63) | 75 | 62.5 |
| MDW | 0. 7730 | 20. 73[20.73, 20. 85) | 69. 12 | 65. 63 |
| Lymph Index | 0. 7860 | 11. 3 [11. 3, 11. 33) | 85. 29 | 50 |
| Lymph Index | 0. 7860 | 11. 63 [11. 63, 11. 72) | 79.41 | 59. 38 |
| Lymph Index | 0. 7860 | 11.8[11.8, 11. 88) | 75 | 65. 63 |
| Lymph Index | 0. 7860 | 12.04[12.04, 12. 07) | 69. 12 | 68. 75 |

### Discussion

Circulating monocytes and lymphocytes are among the first to respond to viral infection. Several previous studies have shown that the volumetric parameters of mononuclear cells are significantly increased during various viral infections [5-7]. Therefore, volumetric increases in these immune cells have potential as viral biomarkers in humans. In current study, we have demonstrated, for the first time, lymph index and monocyte distribution width (MDW) were significantly increased in COVID-19 patients. With designated cutoff values for lymph index and MDW, we achieved the sensitivities of 84.4% and 78.1%, respectively in diagnosing COVID-19 infection. In addition, lymph index in combination with MDW demonstrates excellent diagnostic performance (AUC, 0.83), higher than the AUC with MDW alone and the AUC with lymph index alone. This shows that the combination of MDW and lymph index can more truly reflect the possibility of the subject suffering from SARS-CoV-2 infection, compared with MDW or lymph index alone. Furthermore, we show that lymph index and MDW in suspected patients, like confirmed patients were also significantly increased at the time of admission, suggesting similar pathophysiological process. It is noted that, for the 28 suspected patients tested in this example, they all have negative nucleic acid test at the time of admission, however, 25 of them (89%) were subsequently diagnosed with COVID-19 during the course of disease. This illustrates clinical significance that lymph index and MDW can be used as sensitive screening biomarkers to rapidly identify those suspected individuals before nuclear acid confirmation and to develop proper management plan. We did not see any significant changes in neutrophil volumetric parameters, MNV and NDW, at the time of admission. This is consistent with the observations of recent human studies that neutrophils mainly function as first responders during the innate immune response to acute bacterial infection or sepsis [8-10]

The whole blood cell analysis plays an important role in healthcare decision making from diagnosis through therapy and prognosis. Currently, the automated hematology analyzers are able to provide not only total leukocyte counts, but also leukocyte volumetric parameters. However, changes in leukocyte numerical parameters, such as total leukocyte count, tend to be extremely variable and nonspecific. In the case of COVID-19, total leukocytes or lymphocytes may be normal or mildly decreased, providing no definitive information for differential diagnosis. Therefore, clinical usefulness of volumetric parameters, lymph index and MDW, offers additional practical advantages. These parameters are generated during automated differential analysis without additional specimen requirements. They can be quantitative and are more accurate since significantly more leukocytes are simultaneously evaluated. Furthermore, they offer more robust turn-around-time and are more cost-effective. These volumetric parameters certainly have potential to become useful viral biomarkers to help healthcare providers in the out-patient department or fever clinic to rapidly identify those who might be infected with COVID-19 and to provide valuable information for triage decision making.

The scope of the invention is not limited by the specific embodiments described herein. In fact, from the foregoing description and drawings, various modifications of the present invention other than those described herein will be apparent to those skilled in the art. Such modifications are intended to fall within the scope of the appended claims. Furthermore, all embodiments described herein are considered to be broadly applicable and can be combined with any and all other consistent embodiments as appropriate.

### References

[1] Huang C, Wang Y, Li X, Ren L, Zhao J, Hu Y, Zhang L et al. Clinical features of patients infected with 2019 novel coronavirus in Wuhan, China. Lancet 2020; 395: 497-506
[2] Pan Y, Guan H, Zhou S, Wang Y, Li Q, Zhu T et al. Initial CT findings and temporal changes in patients with the novel coronavirus pneumonia (2019-nCoV): a study of 63 patients in Wuhan, China. European Radiology . 2020. s00330-020-06731-x
[3] Chan JF, Yuan S, Kok KH et al. A familial cluster of pneumonia associated with the 2019 novel coronavirus indicating person to person transmission: a study of a family cluster. Lancet .2020,395(10223):514-523
[4] Tan W, Zhao X, Ma X et al. A novel coronavirus genome identified in a cluster of pneumonia cases-Wuhan, China 2019-2020. N Engl J Med. 2020 Feb 20;382(8):727-733
[5] Zhu Y, Cao X, Xu D. Detection of Morphologic Changes in Peripheral Mononuclear Cells in Hepatitis B Virus Infection Using the Beckman Coulter DXH 800. Laboratory Hematology 2011,17:22-26
[6] JUNG YJ, KIM JH, PARK IR et al. Evaluation of cell population data on the UniCel DxH 800 Coulter Cellular Analysis system as a screening for viral infection in children. International Journal of Laboratory Hematology. 2012, 34, 283-289
[7] Zhu Y, Cao X, Tao G et al. The lymph index: a potential hematological parameter for viral infection. International Journal of Infectious Diseases 2013,17: e490-e493
[8] Chaves F, Tierno B, Xu D. Quantitative determination of neutrophil VCS parameters by the Coulter automated hematology analyzer: new and reliable indicators for acute bacterial infection. American Journal of Clinical Pathology 2005; 124: 440-4.
[9] Chaves F, Tierno B, Xu D. Neutrophil volume distribution width: a new automated hematologic parameter for acute infection. Archive Pathology and Laboratory Medicine 2006; 130: 378-380.
[10] Xu D. Clinical applications of leukocyte morphological parameters. Int J Clin Res. 2015; 1:1.
[11] FRATER JOHN L. ET AL: "COVID-19 and the clinical hematology laboratory",INTERNATIONAL JOURNAL OF LABORATORY HEMATOLOGY,vol. 42, no. S1 20 April 2020 (2020-04-20), pages 11-18, XP055816538,GB; US ISSN: 1751-5521, DOI: 10.1111/ ijlh.13229Retrieved from the Internet:URL:https://onlinelibrary.wiley.com/doi/full-xml/10.1111/ ijlh.13229.

## Claims

1. A method for identifying a subject having a viral infection, wherein the viral infection is Coronavirus infection, the method comprising:
1) flowing a body fluid sample obtained from the subject through a flowcell;
2) measuring individual cells of the plurality of cells in the body fluid sample, wherein the measuring comprises measuring one or more of volume parameter, conductivity parameter, and light scatter parameter, or light scatter and direct current impedance;
3) determining cell population parameter data values in the body fluid sample based on the measuring, wherein the cell population data comprises monocyte distribution width (MDW) and lymph index; and
4) determining whether or not at least one of the one or more cell population data values exceeds a predetermined threshold,
wherein if the values of the MDW and the lymph index are both outside of their respective reference ranges, the Coronavirus infection in the subject is indicated,
wherein the lymph index is calculated according to: lymph index = lymphocyte volume (LV) × lymphocyte distribution width (LV-SD) / lymphocyte conductivity (LC).

2. The method of claim 1, wherein the Coronavirus is SARS-CoV-2.

3. The method of claim 1, wherein if the value of the MDW is greater than:
a) 20.27;
b) 20.42;
c) 20.73; or
d) 21.24,
the viral infection in the subject is indicated.

4. The method of claim 1, wherein if the value of the lymph index is greater than
i) 11.3;
ii) 11.63;
iii) 11.8;
iv) 12.04; or
v) 12.35,
the viral infection in the subject is indicated.

5. The method of claim 1, wherein the body fluid sample is whole blood.

6. The method of claim 1, wherein the subject is:
a) an individual not confirmed of viral infection;
b) an individual suspected of viral infection;
c) negative for viral nuclear acid testing; and/or
d) human.

7. A device for identifying a subject having a viral infection, wherein the viral infection is Coronavirus infection, the device comprising:
1) a transducer for measuring cells passing through a flowcell, wherein the measuring comprises measuring one or more of volume parameter, conductivity parameter, and light scatter parameter, or light scatter and direct current impedance from the cells, and
2) one or more processors configured to perform operations comprising:
receiving and processing measurement data from the transducer,
determining cell population data values based on the measuring, wherein the cell population data comprises monocyte distribution width (MDW) and lymph index, and
determining whether or not at least one of the one or more cell population data values exceeds a predetermined threshold,
wherein if the values of the MDW and the lymph index are both outside of their respective reference ranges, the Coronavirus infection in the subject is indicated,
wherein the lymph index is calculated according to: lymph index = LV × (LV-SD) / LC.

8. The device of claim 7, wherein the Coronavirus is SARS-CoV-2.

9. The device of claim 7, wherein if the value of the MDW is greater than
a) 20.27;
b) 20.42;
c) 20.73; or
d) 21.24,
the viral infection in the subject is indicated.

10. The device of claim 7, wherein if the value of the lymph index is greater than
i) 11.3;
ii) 11.63;
iii) 11.8;
iv) 12.04; or
v) 12.35,
the viral infection in the subject is indicated.

11. The device of claim 7, wherein the subject is:
a) an individual not confirmed of viral infection;
b) an individual suspected of viral infection;
c) negative for viral nuclear acid testing; and/or
d) human.

12. A computer storage media recording an executable program for identifying a subject having a viral infection, wherein the viral infection is Coronavirus infection, **characterized in that** when the executable program is executed by a processor, the following steps are performed:
1) measuring cells passing through a flowcell, wherein the measuring comprises measuring one or more of volume parameter, conductivity parameter, and light scatter parameter, or light scatter and direct current impedance from the cells, and
2) determining cell population parameter data values in the body fluid sample based on the measuring, wherein the cell population data comprises monocyte distribution width (MDW) and lymph index; and
3) determining whether or not at least one of the one or more cell population data values exceeds a predetermined threshold,
wherein if the values of the MDW and the lymph index are both outside of their respective reference ranges, the Coronavirus infection in the subject is indicated,
wherein the lymph index is calculated according to: lymph index = LV × (LV-SD) / LC.

13. The computer storage media of claim 12, wherein the Coronavirus is SARS-CoV-2.

14. The computer storage media of claim 12, wherein if the value of the MDW is greater than
a) 20.27;
b) 20.42;
c) 20.73; or
d) 21.24,
the viral infection in the subject is indicated.

15. The computer storage media of claim 12, wherein if the value of the lymph index is greater than
i) 11.3;
ii) 11.63;
iii) 11.8;
iv) 12.04; or
v) 12.35,
the viral infection in the subject is indicated.

16. The computer storage media of claim 12, wherein the subject is:
a) an individual not confirmed of viral infection;
b) an individual suspected of viral infection;
c) negative for viral nuclear acid testing; and/or
d) human.

## Patentansprüche

1. Verfahren zur Identifizierung einer Person, die eine Virusinfektion aufweist, wobei es sich bei der Virusinfektion um eine Coronavirus-Infektion handelt, wobei das Verfahren umfasst:
1) Durchleiten einer von der Person erhaltenen Körperflüssigkeitsprobe durch eine Durchflusszelle;
2) Messen einzelner Zellen aus der Vielzahl von Zellen in der Körperflüssigkeitsprobe, wobei das Messen das Messen eines oder mehrerer von einem Volumenparameter, einem Leitfähigkeitsparameter und einem Lichtstreuungsparameter oder einer Lichtstreuung und einer Gleichstromimpedanz umfasst;
3) Bestimmen von Zellpopulationsparameter-Datenwerten in der Körperflüssigkeitsprobe auf der Grundlage der Messung, wobei die Zellpopulationsdaten eine Monozytenverteilungsbreite (MDW) und einen Lymphindex umfassen; und
4) Bestimmen, ob mindestens einer von dem einen oder den mehreren Zellpopulationsdatenwerten einen vorbestimmten Schwellenwert überschreitet oder nicht,
wobei, wenn die Werte der MDW und des Lymphindexes beide außerhalb ihrer jeweiligen Referenzbereiche liegen, dies auf die Coronavirus-Infektion bei der Person hinweist,
wobei der Lymphindex berechnet wird nach: Lymphindex = Lymphozytenvolumen (LV) × Lymphozytenverteilungsbreite (LV-SD) / Lymphozytenleitfähigkeit (LC).

2. Verfahren nach Anspruch 1, wobei es sich bei dem Coronavirus um SARS-CoV-2 handelt.

3. Verfahren nach Anspruch 1, wobei, wenn der Wert der MDW größer ist als:
a) 20,27;
b) 20,42;
c) 20,73; oder
d) 21,24,
dies auf die Virusinfektion bei der Person hinweist.

4. Verfahren nach Anspruch 1, wobei, wenn der Wert des Lymphindex größer ist als
i) 11,3;
ii) 11,63;
iii) 11,8;
iv) 12,04; oder
v) 12,35,
dies auf die Virusinfektion bei der Person hinweist.

5. Verfahren nach Anspruch 1, wobei die Körperflüssigkeitsprobe Vollblut ist.

6. Verfahren nach Anspruch 1, wobei die Person:
a) eine Person ist, bei der keine Virusinfektion bestätigt wurde;
b) eine Person ist, bei der der Verdacht auf eine Virusinfektion besteht;
c) beim Virus-Nukleinsäure-Test negativ getestet wurde; und/oder
d) ein Mensch ist.

7. Vorrichtung zur Identifizierung einer Person, die eine Virusinfektion aufweist, wobei es sich bei der Virusinfektion um eine Coronavirus-Infektion handelt, die Vorrichtung umfassend:
1) einen Wandler zum Messen von Zellen, die eine Durchflusszelle passieren, wobei das Messen das Messen eines oder mehrerer von einem Volumenparameter, einem Leitfähigkeitsparameter und einem Lichtstreuungsparameter oder einer Lichtstreuung und einer Gleichstromimpedanz von den Zellen umfasst, und
2) einen oder mehrere Prozessoren, die so ausgebildet sind, dass sie Operationen durchführen, umfassend:
Empfangen und Verarbeiten von Messdaten von dem Wandler,
Bestimmen von Zellpopulationsdatenwerten auf der Grundlage der Messung, wobei die Zellpopulationsdaten eine Monozytenverteilungsbreite (MDW) und einen Lymphindex umfassen, und
Bestimmen, ob mindestens einer von dem einen oder den mehreren Zellpopulationsdatenwerten einen vorbestimmten Schwellenwert überschreitet oder nicht,
wobei, wenn die Werte der MDW und des Lymphindexes beide außerhalb ihrer jeweiligen Referenzbereiche liegen, dies auf die Coronavirus-Infektion bei der Person hinweist,
wobei der Lymphindex berechnet wird nach: Lymphindex = LV × (LV-SD) /LC.

8. Vorrichtung nach Anspruch 7, wobei es sich bei dem Coronavirus um SARS-CoV-2 handelt.

9. Vorrichtung nach Anspruch 7, wobei, wenn der Wert der MDW größer ist als
a) 20,27;
b) 20,42;
c) 20,73; oder
d) 21,24,
dies auf die Virusinfektion bei der Person hinweist.

10. Vorrichtung nach Anspruch 7, wobei, wenn der Wert des Lymphindex größer ist als
i) 11,3;
ii) 11,63;
iii) 11,8;
iv) 12,04; oder
v) 12,35,
dies auf die Virusinfektion bei der Person hinweist.

11. Vorrichtung nach Anspruch 7, wobei die Person:
a) eine Person ist, bei der keine Virusinfektion bestätigt wurde;
b) eine Person ist, bei der der Verdacht auf eine Virusinfektion besteht;
c) beim Virus-Nukleinsäure-Test negativ getestet wurde; und/oder
d) ein Mensch ist.

12. Computerspeichermedium, auf dem ein ausführbares Programm zur Identifizierung einer Person, die eine Virusinfektion aufweist, aufgezeichnet ist, wobei es sich bei der Virusinfektion um eine Coronavirus-Infektion handelt, **dadurch gekennzeichnet, dass** bei Ausführung des ausführbaren Programms durch einen Prozessor die folgenden Schritte durchgeführt werden:
1) Messen von Zellen, die eine Durchflusszelle passieren, wobei das Messen das Messen von einem oder mehreren von einem Volumenparameter, einem Leitfähigkeitsparameter und einem Lichtstreuungsparameter oder einer Lichtstreuung und einer Gleichstromimpedanz von den Zellen umfasst, und
2) Bestimmen von Zellpopulationsparameter-Datenwerten in der Körperflüssigkeitsprobe auf der Grundlage des Messens, wobei die Zellpopulationsdaten eine Monozytenverteilungsbreite (MDW) und einen Lymphindex umfassen; und
3) Bestimmen, ob mindestens einer von dem einen oder den mehreren Zellpopulationsdatenwerten einen vorbestimmten Schwellenwert überschreitet oder nicht,
wobei, wenn die Werte der MDW und des Lymphindexes beide außerhalb ihrer jeweiligen Referenzbereiche liegen, dies auf die Coronavirus-Infektion bei der Person hinweist,
wobei der Lymphindex berechnet wird nach: Lymphindex = LV × (LV-SD) / LC.

13. Computerspeichermedium nach Anspruch 12, wobei es sich bei dem Coronavirus um SARS-CoV-2 handelt.

14. Computerspeichermedium nach Anspruch 12, wobei, wenn der Wert der MDW größer ist als
a) 20,27;
b) 20,42;
c) 20,73; oder
d) 21,24,
dies auf die Virusinfektion bei der Person hinweist.

15. Computerspeichermedium nach Anspruch 12, wobei, wenn der Wert des Lymphindex größer ist als
i) 11,3;
ii) 11,63;
iii) 11,8;
iv) 12,04; oder
v) 12,35,
dies auf die Virusinfektion bei der Person hinweist.

16. Computerspeichermedium nach Anspruch 12, wobei die Person:
a) eine Person ist, bei der keine Virusinfektion bestätigt wurde;
b) eine Person ist, bei der der Verdacht auf eine Virusinfektion besteht;
c) beim Virus-Nukleinsäure-Test negativ getestet wurde; und/oder
d) ein Mensch ist.

## Revendications

1. Procédé pour identifier un sujet présentant une infection virale, dans lequel l'infection virale est une infection à coronavirus, le procédé comprenant ce qui suit :
1) faire s'écouler un échantillon de fluide corporel obtenu à partir du sujet à travers une cellule d'écoulement ;
2) mesurer des cellules individuelles de la pluralité de cellules dans l'échantillon de fluide corporel, dans lequel la mesure comprend le fait de mesurer un ou plusieurs parmi : paramètre de volume, paramètre de conductivité et paramètre de diffusion de la lumière, ou diffusion de la lumière et impédance en courant continu ;
3) déterminer, sur la base de la mesure, des valeurs de données de paramètres de population cellulaire dans l'échantillon de fluide corporel, dans lequel les données de population cellulaire comprennent la largeur de distribution des monocytes (MDW) et l'indice lymphatique ; et
4) déterminer si oui ou non au moins une desdites une ou plusieurs valeurs de données de population cellulaire dépasse un seuil prédéterminé,
dans lequel, si les valeurs du MDW et de l'indice lymphatique sont toutes deux en dehors de leurs intervalles de référence respectifs, l'infection à coronavirus chez le sujet est indiquée,
dans lequel l'indice lymphatique est calculé comme suit : indice lymphatique = volume lymphocytaire (LV) x largeur de distribution des lymphocytes (LV-SD) / conductivité lymphocytaire (LC).

2. Procédé selon la revendication 1, dans lequel le coronavirus est SARS-CoV-2.

3. Procédé selon la revendication 1, dans lequel, si la valeur de la MDW est supérieure à :
a) 20,27 ;
b) 20,42 ;
c) 20,73 ; ou
d) 21,24,
l'infection virale chez le sujet est indiquée.

4. Procédé selon la revendication 1, dans lequel, si la valeur de l'indice lymphatique est supérieure à
i) 11,3 ;
ii) 11,63 ;
iii) 11,8 ;
iv) 12,04 ; ou
v) 12,35,
l'infection virale chez le sujet est indiquée.

5. Procédé selon la revendication 1, dans lequel l'échantillon de fluide corporel est du sang total.

6. Procédé selon la revendication 1, dans lequel le sujet est :
a) un individu chez lequel une infection virale n'a pas été confirmée ;
b) un individu suspecté d'infection virale ;
c) négatif au test d'acides nucléiques viraux ; et/ou
d) humain.

7. Dispositif pour identifier un sujet présentant une infection virale, dans lequel l'infection virale est une infection à coronavirus, le dispositif comprenant :
1) un transducteur destiné à mesurer des cellules passant à travers une cellule d'écoulement, dans lequel la mesure comprend le fait de mesurer un ou plusieurs parmi : paramètre de volume, paramètre de conductivité, et paramètre de diffusion de la lumière, ou diffusion de la lumière et impédance en courant continu à partir des cellules, et
2) un ou plusieurs processeurs configurés pour effectuer des opérations comprenant :
le fait de recevoir et de traiter des données de mesure provenant du transducteur,
le fait, sur la base de la mesure, de déterminer des valeurs de données de population cellulaire, dans lequel les données de population cellulaire comprennent la largeur de distribution des monocytes (MDW) et l'indice lymphatique, et
le fait de déterminer si oui ou non au moins une desdites une ou plusieurs valeurs de données de population cellulaire dépasse un seuil prédéterminé,
dans lequel, si les valeurs du MDW et de l'indice lymphatique sont toutes deux en dehors de leurs intervalles de référence respectifs, l'infection à coronavirus chez le sujet est indiquée,
dans lequel l'indice lymphatique est calculé comme suit : indice lymphatique = LV x (LV-SD)/LC.

8. Dispositif selon la revendication 7, dans lequel le coronavirus est SARS-CoV-2.

9. Dispositif selon la revendication 7, dans lequel, si la valeur de la MDW est supérieure à
a) 20,27 ;
b) 20,42 ;
c) 20,73 ; ou
d) 21,24,
l'infection virale chez le sujet est indiquée.

10. Dispositif selon la revendication 7, dans lequel, si la valeur de l'indice lymphatique est supérieure à
i) 11,3 ;
ii) 11,63 ;
iii) 11,8 ;
iv) 12,04 ; ou
v) 12,35,
l'infection virale chez le sujet est indiquée.

11. Dispositif selon la revendication 7, dans lequel le sujet est :
a) un individu chez lequel une infection virale n'a pas été confirmée ;
b) un individu suspecté d'infection virale ;
c) négatif au test d'acides nucléiques viraux ; et/ou
d) humain.

12. Support de stockage informatique enregistrant un programme exécutable pour identifier un sujet présentant une infection virale, dans lequel l'infection virale est une infection à coronavirus, **caractérisé en ce que** lorsque le programme exécutable est exécuté par un processeur, les étapes suivantes sont mises en œuvre :
1) mesurer des cellules passant à travers une cellule d'écoulement, dans lequel la mesure comprend le fait de mesurer un ou plusieurs parmi : paramètre de volume, paramètre de conductivité, et paramètre de diffusion de la lumière, ou diffusion de la lumière et impédance en courant continu à partir des cellules, et
2) déterminer, sur la base de la mesure, des valeurs de données de paramètres de population cellulaire dans l'échantillon de fluide corporel, dans lequel les données de population cellulaire comprennent la largeur de distribution des monocytes (MDW) et l'indice lymphatique ; et
3) déterminer si oui ou non au moins une desdites une ou plusieurs valeurs de données de population cellulaire dépasse un seuil prédéterminé,
dans lequel, si les valeurs du MDW et de l'indice lymphatique sont toutes deux en dehors de leurs intervalles de référence respectifs, l'infection à coronavirus chez le sujet est indiquée,
dans lequel l'indice lymphatique est calculé comme suit : indice lymphatique = LV x (LV-SD)/LC.

13. Support de stockage informatique selon la revendication 12, dans lequel le coronavirus est SARS-CoV-2.

14. Support de stockage informatique selon la revendication 12, dans lequel, si la valeur de la MDW est supérieure à
a) 20,27 ;
b) 20,42 ;
c) 20,73 ; ou
d) 21,24,
l'infection virale chez le sujet est indiquée.

15. Support de stockage informatique selon la revendication 12, dans lequel, si la valeur de l'indice lymphatique est supérieure à
i) 11,3 ;
ii) 11,63 ;
iii) 11,8 ;
iv) 12,04 ; ou
v) 12,35,
l'infection virale chez le sujet est indiquée.

16. Support de stockage informatique selon la revendication 12, dans lequel le sujet est :
a) un individu chez lequel une infection virale n'a pas été confirmée ;
b) un individu suspecté d'infection virale ;
c) négatif au test d'acides nucléiques viraux ; et/ou
d) humain.
